# DEMANDE DE BREVET EUROPEEN

(11) **EP 4 397 300 A1**
(43) Date de publication de la demande: **10.07.2024**
(21) Numéro de dépôt: 23307278.4
(22) Date de dépôt: 20.12.2023
(51) Int. Cl.: A61K 8/9722, A61K 8/9789, A61Q 1/02, A61Q 1/10, A61K 8/19

(54) **ASSOCIATION COMPRENANT UN EXTRAIT DE SPIRULINE OU DE PIGMENT INDIGO ET UN PIGMENT D'OXYDE DE FER NOIR, ET UTILISATIONS**

(30) Priorité: 23.12.2022 FR 2214398; 02.02.2023 FR 2300987; 20.04.2023 FR 2303967
(71) Demandeur: Laboratoires De Biologie Vegetale Yves Rocher, 56200 La Gacilly (FR)
(72) Inventeur: SEBBAN, Sarah, 92330 Sceaux (FR); OPEL, Caroline, 78220 Viroflay (FR)
(74) Mandataire: Novagraaf Technologies

(57) **Abrégé**

La présente invention se rapporte à une utilisation cosmétique non thérapeutique d'une combinaison d'extrait de spiruline et/ou d'un pigment indigo et de pigment d'oxyde de fer noir pour apporter de l'éclat au teint de la peau et/ou des muqueuses.

La présente invention se rapporte également à une utilisation cosmétique non thérapeutique d'un extrait de spiruline et/ou de pigment indigo dans une composition cosmétique ou dermatologique comprenant un pigment d'oxyde de fer noir, pour diminuer un effet de ternissement de la teinte de ladite composition cosmétique ou dermatologique dû à la présence du pigment d'oxyde de fer noir, ou pour rendre une coloration noire plus profonde.

La présente invention se rapporte en outre à une association comprenant un extrait de spiruline et/ou de pigment indigo et un pigment d'oxyde de fer noir, dans laquelle le ratio entre la quantité d'extrait de spiruline ou d'extrait d'indigo et la quantité d'oxyde de fer noir est comprise entre 1/99 et 99/1, de préférence environ 100/0, la valeur de 0 étant possiblement exclue.

## Description

### Domaine technique de l'invention

La présente invention se rapporte à une utilisation cosmétique non thérapeutique d'une combinaison d'extrait de spiruline et/ou de pigment indigo et de pigment d'oxyde de fer noir, ainsi qu'à une association comprenant un extrait de spiruline et/ou de pigment indigo et un pigment d'oxyde de fer noir.

La présente invention trouve une application notamment dans le domaine des cosmétiques.

### Art antérieur

Les produits cosmétiques destinés au maquillage de la peau et des lèvres visent à procurer un effet esthétique, notamment en apportant une coloration plus ou moins prononcée, voire un effet de camouflage à l'égard des défauts cutanés.

Pour procurer de tels effets, les produits cosmétiques associent généralement des charges minérales ou organiques et des agents de coloration.

Les charges permettent de conférer du corps et/ou de la douceur au produit cosmétique, et de l'uniformité au maquillage.

En ce qui concerne les agents de coloration, ils sont plus particulièrement utilisés à titre d'opacifiant et/ou de colorant et sont généralement présents à une concentration suffisante pour procurer la couleur souhaitée.

Les agents de coloration les plus fréquemment utilisés sont les pigments. Il s'agit généralement de particules blanches ou colorées, minérales ou organiques, insolubles dans la phase hydrophile ou lipophile liquide, destinées à colorer et/ou à opacifier le produit cosmétique, la peau ou les muqueuses.

Les pigments d'oxydes de fer noir sont connus pour leurs propriétés couvrantes et colorantes superficielles et momentanées sur la peau. Ils sont généralement utilisés dans les produits de maquillage tels que les fonds de teint, les mascara et, les eye-liners. Toutefois, il peut être difficile d'apporter de l'éclat à la peau en utilisant ces produits car ceux-ci ont tendance à procurer un effet grisâtre, ce qui rend le teint terne après application sur la peau, et la couleur peu profonde.

Il existe donc un réel besoin de produits cosmétiques palliant ces défauts, inconvénients et obstacles de l'art antérieur, en particulier permettant d'obtenir une coloration soutenue et/ou éviter de ternir le teint après application de pigments d'oxyde de fer sur la peau ou les muqueuses.

### Exposé de l'invention

La présente invention a précisément pour but de répondre à ces besoins et inconvénients de l'art antérieur.

Les inventeurs ont en outre mis au point une association comprenant un extrait de spiruline et/ou de pigment indigo et un pigment d'oxyde de fer noir, pour répondre à ces besoins, et notamment apporter de l'éclat au teint de la peau et/ou des muqueuses.

Les inventeurs ont de plus mis en évidence qu'un extrait de spiruline et/ou de pigment indigo permet, dans une composition cosmétique ou dermatologique comprenant un pigment d'oxyde de fer noir, de diminuer un effet de ternissement de la teinte de la composition dû à la présence du pigment d'oxyde de fer noir, et de rendre une coloration noire plus profonde, ce qui est particulièrement avantageux pour une utilisation dans un mascara ou un eye-liner.

Ainsi, un premier objet de l'invention se rapporte à une utilisation cosmétique non thérapeutique d'une combinaison d'extrait de spiruline et/ou de pigment indigo et de pigment d'oxyde de fer noir pour apporter de l'éclat au teint de la peau et/ou des muqueuses.

Un deuxième objet de l'invention se rapporte à une utilisation cosmétique non thérapeutique d'un extrait de spiruline et/ou de pigment indigo dans une composition cosmétique ou dermatologique comprenant un pigment d'oxyde de fer noir, pour augmenter la profondeur de la teinte noire de ladite composition cosmétique ou dermatologique dû à la présence du pigment d'oxyde de fer noir.

Un troisième objet de l'invention se rapporte à une association comprenant un extrait de spiruline et/ou de pigment indigo et un pigment d'oxyde de fer noir, dans laquelle le ratio entre la quantité d'extrait de spiruline ou d'extrait d'indigo et la quantité d'oxyde de fer noir est comprise entre 99/1 et 1/99 de préférence environ 100/0, la valeur de 0 étant possiblement exclue.

Un quatrième objet de l'invention se rapporte à une composition cosmétique ou dermatologique comprenant l'association, et au moins un véhicule cosmétiquement et/ou dermatologiquement acceptable.

Enfin, un cinquième objet de l'invention se rapporte à un dispositif se présentant sous une forme choisie parmi un pot, un flacon-pompe, une lingette, un masque, un patch, un spray, un stick et une flaconnette, ledit dispositif comprenant l'association, ou la composition cosmétique ou dermatologique.

La spiruline est une cyanobactérie filamenteuse de couleur bleue verte. Ses bienfaits sur la santé lui valent une popularité d'envergure internationale. On l'utilise dans le domaine alimentaire sous forme de poudre, de paillettes, de comprimés ou encore en gélules. La spiruline est considérée comme un super-aliment en raison de ses propriétés nutritionnelles uniques au monde, ainsi que ses propriétés anti-oxydante, anti-inflammatoire et anti-âge. En outre, elle est l'une des meilleures sources de protéines végétales qui soient, en plus d'être une exceptionnelle source de fer. Dans le cadre de l'invention, toute espèce de spiruline peut être utilisée, comme Spirulina Platensis et/ou Spirulina Maxima. L'origine de la spiruline utilisée n'est pas particulièrement limitée, et des produits naturels ou des cultures peuvent être utilisés de manière appropriée.

Un « extrait de spiruline », au sens de la présente invention, peut être tout extrait de couleur bleue permettant d'obtenir les effets mentionnés ci-avant. Il peut s'agir d'un extrait total obtenu par cryobroyage, d'un extrait obtenu par broyage et séchage, d'un extrait obtenu par pression, d'un extrait obtenu par action d'un solvant, d'un extrait issu d'un procédé enzymatique ou par lyse cellulaire. L'extrait peut être insoluble ou très partiellement soluble.

Avantageusement, l'extrait de spiruline possède en outre des propriétés d'origine anti-oxydant, anti-inflammatoire et anti-âge, particulièrement avantageuses pour une utilisation cosmétique.

L'indigo est une matière colorante bleu-violacé dont l'usage le plus ancien est la teinture de vêtements. Cette matière est traditionnellement extraite des feuilles et/ou des tiges de diverses plantes tinctoriales, dont l'indigotier (Indigofera Tinctoria), le pastel des teinturiers en Europe, la renouée des teinturiers en Chine (extraite de feuilles de Persicaire, également appelé Polygonum Tinctorium ou Persicaria tinctoria), le Strobilanthes cusia en Asie du Sud-Est, le gara Philenoptera cyanescens en Afrique. Divers procédés de manufacture furent également développés, permettant d'obtenir des indigos synthétiques comme le bleu d'indanthrone (BASF). Dans le cadre de l'invention, un indigo de préférence naturel peut être utilisé. De préférence, l'indigo utilisé est un pigment naturel issu des feuilles et/ou tiges de l'indigotier (Indigofera Tinctoria) ou issu des feuilles, éventuellement fraîches, et/ou tiges du Persicaire (Polygonum Tinctorium Leaf/Stem extract).

Les pigments d'oxyde de fer noir sont des pigments naturels ou synthétiques, à haut pouvoir colorant, traditionnellement utilisés pour obtenir un noir bleuté ou un noir plus profond. En cosmétique, le pigment utilisé est le tétraoxyde de fer (nom INCI : Cl 77499), présent dans les fonds de teint, les mascaras, et les eye-liners. Ils permettent de foncer d'autres couleurs et d'agrandir les palettes de couleurs en créant des dégradés, notamment plus ou moins foncés, noirs, saturés ou grisés.

On entend par « peau », au sens de la présente invention, une peau claire, de phototype I (type celte), Il (type germanique), III (type européen) ou IV (type méditerranéen).

On entend par « apporter de l'éclat au teint », au sens de la présente invention, le fait de rendre la peau plus lumineuse et/ou d'obtenir un teint moins terne et/ou plus radiant. Avantageusement, l'indice de radiance d'une composition cosmétique comprenant un extrait de spiruline et/ou d'indigo est supérieur à une composition cosmétique ne comprenant pas un tel extrait. Cet effet peut être mesuré par toute méthode connue, par exemple au moyen d'un goniospectrophotomètre multi-angles.

On entend par « diminuer un effet de ternissement de la teinte », au sens de la présente invention, le fait de rendre une composition cosmétique ou dermatologique plus lumineuse, moins terne, moins grisâtre, moins olive, plus « glowy » (plus éclatante ou radiante) par rapport à la même composition ne comprenant pas d'extrait de spiruline et/ou de pigment indigo. Ici encore, cet effet peut être mesuré par toute méthode connue, par exemple au moyen d'un goniospectrophotomètre multi-angles.

On entend par « rendre une coloration noire plus profonde », au sens de la présente invention, le fait d'obtenir une couleur noire plus intense, avec pas ou peu de reflet, par rapport à la même composition ne comprenant pas d'extrait de spiruline et/ou de pigment indigo. Cet effet est avantageux notamment dans le domaine des mascaras et des eye-liner. Cet effet peut être mesuré par toute méthode connue, par exemple au moyen d'un goniospectrophotomètre.

Avantageusement, dans l'association de l'invention, le ratio entre la quantité d'extrait de spiruline et/ou d'extrait d'indigo et la quantité d'oxyde de fer noir peut être de 1/99, ou de 5/95, ou de 10/90, ou de 20/80, ou de 30/70, ou de 40/60, ou de 50/50, ou de 60/40, ou de 70/30, ou de 80/20, ou de 90/10, ou de 95/5, ou de 99/1, ou de 100/0, la valeur de 0 étant exclue.

Avantageusement, dans la composition de l'invention, le pourcentage en poids de l'extrait de spiruline et/ou de pigment indigo peut être compris entre 0,0001 et 11,250%, en poids, et le pourcentage en poids d'oxyde de fer noir est compris entre 0,0001 et 15,000%, en poids, par rapport au poids total de la composition.

Avantageusement, lorsque la composition de l'invention est une composition unifiant le teint cutané, par exemple un fond de teint, le pourcentage en poids de l'extrait de spiruline et/ou de pigment indigo peut être compris entre 0,0001 et 2,5%, en poids, et le pourcentage en poids d'oxyde de fer noir est compris entre 0,0001 et 2,5%, en poids, par rapport au poids total de la composition. Dans ce cas, le pourcentage en poids de l'extrait de spiruline et/ou de pigment indigo peut être à ce titre compris entre 0,0001 et 2,5%, ou entre 0,01 et 2,5%, ou entre 0,1 et 2,5%, ou entre 1,0 et 2,0%. Le pourcentage en poids d'oxyde de fer noir peut être compris entre 0,0001 et 2,5%, ou entre 0,01 et 2,5%, ou entre 0,1 et 2,5%, ou entre 1,0 et 2,0%.

Avantageusement, lorsque la composition de l'invention est un mascara ou un eyeliner, le pourcentage en poids de l'extrait de spiruline et/ou de pigment indigo peut être compris entre 0,0001 et 11,250%, en poids par rapport au poids total de la composition, par exemple environ 0,15%, ou environ 0,75% ou environ 1,5% ou environ 3,75%, ou environ 7,5%, ou environ 11,25%. Le pourcentage en poids de l'oxyde de fer noir peut être compris entre 0,0001 et 15,000% en poids par rapport au poids total de la composition, par exemple environ 3,75%, ou environ 7,5%, ou environ 11,25%, ou environ 13,5%, ou environ 14,25%, ou environ 14,85%.

On entend par « composition cosmétique ou dermatologique », dans la présente invention, toute composition à visée cosmétique c'est à dire esthétique, ou pour application cutanée, pouvant être mise en contact avec les parties superficielles du corps humain, par exemple l'épiderme, les systèmes pileux et capillaires, le cuir chevelu et les muqueuses externes. Avantageusement, une composition cosmétique permet, exclusivement ou principalement, de les protéger, parfumer, maintenir en bon état, modifier leur aspect esthétique ou en corriger les défauts superficiels. Notamment, une composition cosmétique peut comprendre au moins un véhicule cosmétiquement ou dermatologiquement acceptable. Avantageusement, la composition peut être choisie parmi une composition unifiant le teint cutané, par exemple un fond de teint, une crème teintée, une BB cream, une poudre de teint, un blush, un concealer, un enlumineur de teint ou un anticernes ; elle peut également être une composition de coloration des cils et/ou des sourcils et/ou une composition soulignant le bord des paupières, ou la partie mobile de la paupière, comme par exemple un fard à paupières. De préférence, la composition peut être choisie parmi une composition unifiant le teint cutané et une composition de coloration des cils et/ou des sourcils.

Par « véhicule cosmétiquement ou dermatologiquement acceptable », on entend au moins un véhicule adapté pour une utilisation en contact avec des cellules humaines cutanées, en particulier les cellules de l'épiderme, sans toxicité, irritation, réponse allergique indue et similaire, et proportionné à un rapport avantage/risque raisonnable. A ce titre, le véhicule peut être tout véhicule adapté à l'obtention d'une composition de forme anhydre, ou de forme d'émulsion eau dans huile, ou de forme émulsion, huile dans eau ou encore d'un gel aqueux.

La composition cosmétique mise en oeuvre dans l'invention peut être obtenue par tout procédé approprié connu de l'homme du métier pour la fabrication d'une composition cosmétique ou dermatologique. Il peut s'agir, par exemple d'un simple mélange. Il peut s'agir également, par exemple, d'un procédé comprenant une étape d'incorporation d'une phase interne dans une phase externe au moyen d'un émulseur, par exemple d'une turbine de type rotor-stator ou une turbine pâle type défloculeuse.

Les compositions selon l'invention peuvent également contenir des ingrédients cosmétiques additionnels classiquement mis en oeuvre pour la formulation de galéniques particulières généralement ajustée au regard de la matière kératinique visée. Ce ou ces ingrédients cosmétiques additionnels peuvent notamment être choisis parmi les cires, les corps gras pâteux, les polymères filmogènes, les tensioactifs non ioniques, anioniques, cationiques, les gélifiants hydrophiles ou lipophiles, les actifs hydrophiles ou lipophiles, les conservateurs, les antioxydants, les solvants, les parfums, les charges, les filtres solaires, les bactéricides, les absorbeurs d'odeur, les matières colorantes additionnelles comme les pigments, nacres, colorants hydrosolubles ou liposolubles, les sels, et leurs mélanges. Les quantités des ingrédients cosmétiques additionnels sont celles classiquement utilisées dans le domaine considéré, et par exemple de 0,01 à 20 % du poids total de la composition, et de préférence de 0,01 à 15 % du poids total de la composition.

La composition de l'invention peut se trouver selon toute forme utilisée en cosmétique, par exemple une crème, un onguent, un baume, un beurre, un lait, une émulsion eau dans huile, une émulsion huile dans eau, un gel aqueux, et un produit anhydre sous forme de gel ou de stick.

D'autres avantages pourront encore apparaître à l'homme du métier à la lecture des exemples ci-dessous, illustrés par les figures annexées, donnés à titre illustratif.

### Brève description des figures

[Fig. 1] représente la mesure du facteur de radiance à angle d'incidence 0° en fonction de l'angle de réflexion, d'une formulation comprenant extrait de spiruline ou d'indigo mélangé à un pigment d'oxyde de fer proportions 50/50 (KADFA25 et KADFA29 respectivement) en comparaison avec une formule équivalente dépourvu d'extrait de spiruline (KADFA28), et avec une formule fond de teint « Le radieux » (Laboratoires de Biologie Végétale Yves Rocher) et du fond de teint Super Mat.
[Fig. 2] représente la mesure du facteur de radiance moyen, en fonction des angles de réflexion (en °), pour différentes formules telles que décrites à l'Exemple 2, de teinte 200 beige correspondant au phototype de type II.
[Fig. 3] représente la mesure du facteur de radiance moyen, en fonction des angles de réflexion (en °), pour différentes formules telles que décrites à l'Exemple 3, de teinte brun 900 correspondant au phototype de type IV.
[Fig. 4] représente la mesure du facteur de radiance moyen, en fonction des angles de réflexion (en °), pour différentes formules telles que décrites à l'Exemple 4, de teinte noir mat.

### EXEMPLES

### Exemple 1 : Augmentation de la radiance (éclat) apportée par une composition comprenant une combinaison d'un extrait de spiruline ou d'indigo et de pigment d'oxyde de fer noir dans un fond de teint beige pour un phototype II

Un extrait de spiruline (Sensient, NATPURE XFINE SPIRULINA SL 615) ou d'indigo (Indigo Naturel de Renouée (Green'Ing) - INCI : Polygonum tinctorium leaf/stem extract) est mélangé à un pigment d'oxyde de fer noir (Sun Chemical, SunPUROTM Black Iron Oxide C33-7001) en proportions égales (50/50), dans une formule de fond de teint de marque « Le Radieux » (Laboratoires de Biologie Végétale Yves Rocher), qui est une émulsion E/H, teinte 200 beige correspondant au phototype de type II).

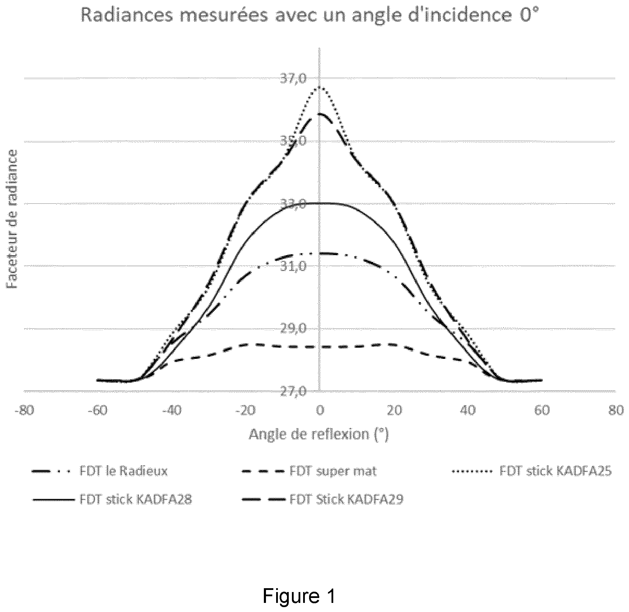

La luminosité (Delta L) de ces formules (notée KADFA25 pour la formule contenant de la spiruline et KADFA29 pour la formule contenant l'indigo sur la figure 1) est comparée par des mesures à un goniospectrophotomètre multi-angles (Seelab GX), à celle d'une formule équivalente mais ne contenant que de l'oxyde de fer noir (notée KADFA28 sur la figure 1), en une quantité égale au double de la quantité d'oxyde de fer noir contenue dans la formule KADFA25, ainsi qu'à celle du fond de teint « Le Radieux » et du fond de teint « Super Mat » (Teinte 200 beige correspondant à un phototype de type II, Laboratoires de Biologie Végétale Yves Rocher).

Les résultats, représentés à la Figure 1, montrent que les formules contenant l'extrait de spiruline ou d'indigo mélangé à un pigment d'oxyde de fer en proportions 50/50 ont un facteur de radiance (confèrent l'éclat à la formule) supérieur à celui des autres formules.

### Exemple 2 : Augmentation de la radiance (éclat) apportée par une composition comprenant une combinaison d'un extrait de pigment bleu et de pigment d'oxyde de fer noir dans un fond de teint beige pour un phototype II

Des associations sont réalisées en des proportions et dans des formules telles que mentionnées dans le tableau I ci-après. L'indice de radiance (« glow ») est comparé dans chaque formule avec celui mesuré pour le fond de teint (FDT) « Le Radieux », qui est comme indiqué dans l'Exemple 1 une émulsion E/H, teinte 200 beige correspondant au phototype de type II. Le pigment issu d' Indigofera Tinctoria est le pigment NC 152 Blue Pigment (Pigm'azur).

**[Table 1]**

| Référé nce | KADFA25 | KADFA28 | KADFA29 | KADFA31 | KADFA32 | KADFA33 | KADFA34 | KADFC03 | KADFC04 | KADFC05 | FDT Le Radieux |
|---|---|---|---|---|---|---|---|---|---|---|---|
| Teinte | 200 beige (phototyp e II) | 200 beige (phototyp e II) | 200 beige (phototyp e II) | 200 beige (phototyp e II) | 200 beige (phototyp e II) | 200 beige (phototyp e II) | 200 beige (phototyp e II) | 200 beige (phototyp e II) | 200 beige (phototyp e II) | 200 beige (phototyp e II) | 200 beige (phototyp e II) |
| Ratio pigmen t | 50/50 spiruline / oxyde fer noir | 100% OX FER NOIR | 50/50 INDIGO / OX FER NOIR | 100% spiruline | 50/50 INDIGO / SPIRULIN E | 75/25 spiruline / oxyde fer noir | 25/75 spiruline / oxyde fer noir | 10/90 spiruline / oxyde fer noir | 5/95 spiruline / oxyde fer noir | 5/95 Indigofera Tinctoria Extract / OXYDE DE FER NOIR | 100% oxyde de fer noir |
| Indice de Glow | supérieur au bench FDT Le Radieux | Légèreme nt supérieur au bench FDT Le Radieux | supérieur au bench FDT Le Radieux | supérieur au bench FDT Le Radieux | supérieur au bench FDT Le Radieux | supérieur au bench FDT Le Radieux | supérieur au bench FDT Le Radieux | supérieur au bench FDT Le Radieux | supérieur au bench FDT Le Radieux | supérieur au bench FDT Le Radieux | NA |
| Type/g aléniqu e | Fond de teint stick/anhy dre | Fond de teint stick/anhy dre | Fond de teint stick/anhy dre | Fond de teint stick/anhy dre | Fond de teint stick/anhy dre | Fond de teint stick/anhy dre | Fond de teint stick/anhy dre | Fond de teint émulsion E/H | Fond de teint émulsion E/H | Fond de teint émulsion E/H | Fond de teint émulsion E/H |

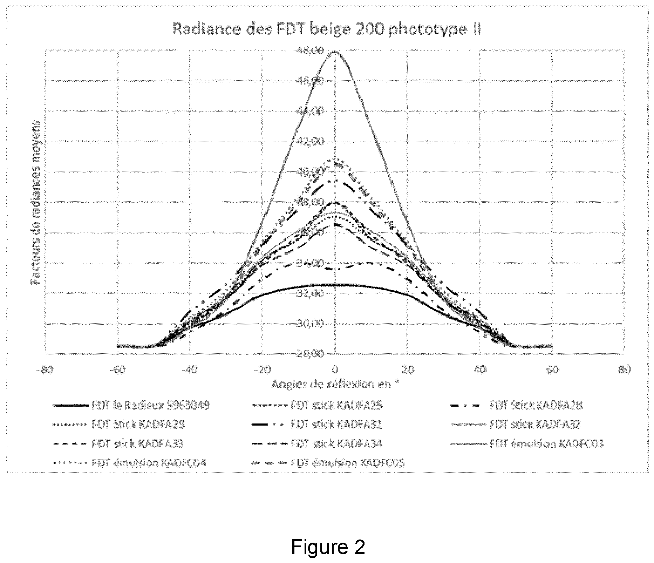

Les mesures présentées dans le tableau I, et représentées en Figure 2, montrent qu'une association de pigment bleu naturel, que ce soit la spiruline ou l'indigo, à l'oxyde de fer noir, permet d'augmenter la radiance d'un fond de teint beige pour phototype II.

### Exemple 3 : Augmentation de la radiance (éclat) apportée par une composition comprenant une combinaison d'un extrait de spiruline et de pigment d'oxyde de fer noir dans un fond de teint brun pour un phototype IV

Des associations sont réalisées en des proportions et dans des formules telles que mentionnées dans le tableau Il ci-après. L'indice de radiance (« glow ») est comparé dans chaque formule avec celui mesuré pour le fond de teint (FDT) « Zéro Défaut » (Laboratoires de Biologie Végétale Yves Rocher), qui est une émulsion E/H, teinte 900 brun correspondant au phototype de type IV.

**[Table 2]**

| Référence | KADFC01 | KADFC02 | FDT Zéro Défaut |
|---|---|---|---|
| Teinte | 900 bruns (phototype IV) | 900 bruns (phototype IV) | 900 bruns (phototype IV) |
| Ratio pigment | 50/50 SPIRULINE / OX FER NOIR | 75/25 SPIRULINE / OX FER NOIR | 100% oxyde de fer noir |
| Indice de Glow | supérieur au bench FDT Zéro Défaut | supérieur au bench FDT Zéro Défaut | NA |
| Type/galé nique | Fond de teint émulsion E/H | Fond de teint émulsion E/H | Fond de teint émulsion E/H |

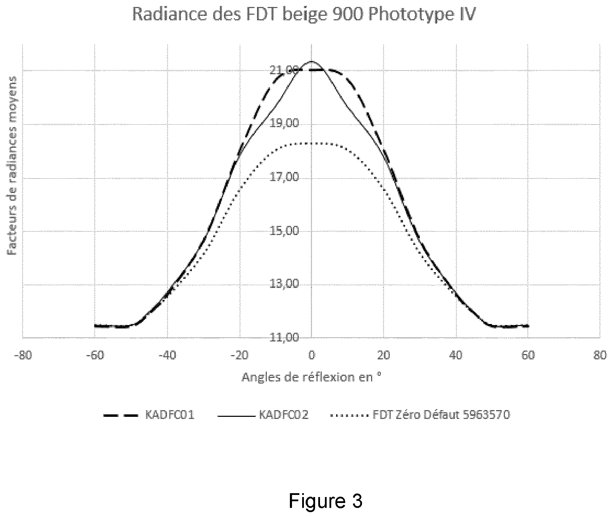

Les mesures présentées dans le tableau 2, et représentées en Figure 3, montrent qu'une association de spiruline à l'oxyde de fer noir, permet d'augmenter la radiance d'un fond de teint brun pour phototype IV.

### Exemple 4 : Augmentation de la profondeur apportée par une composition comprenant une combinaison d'un extrait de spiruline et de pigment d'oxyde de fer noir dans un eyeliner

Des associations sont réalisées en des proportions et dans des formules telles que mentionnées dans le tableau III ci-après. L'indice de radiance (« glow ») est comparé dans chaque formule avec celui mesuré pour le produit « Eye liner mat » (Laboratoires de Biologie Végétale Yves Rocher), qui est un gel aqueux.

**[Table 3]**

| Référence | KADFB05 | KADFB06 | Eye liner mat |
|---|---|---|---|
| Teinte | NOIR (mat) | NOIR (mat) | NOIR (mat) |
| Ratio pigment | 50/50 OXYDE DE FER NOIR/ SPIRULINE | 100% SPIRULINE | 100% oxyde de fer noir |
| Indice de Glow | supérieur au bench Eye Liner Mat | supérieur au bench Eye Liner Mat | NA |
| Type/galé nique | Gel aqueux | Gel aqueux | Gel aqueux |

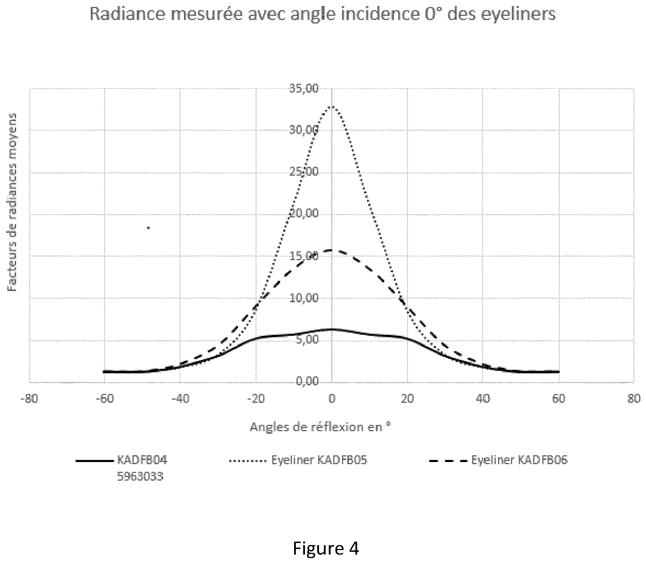

Les mesures présentées dans le tableau 3, et représentées en Figure 4, montrent qu'une association de pigment spiruline à l'oxyde de fer noir, permet d'augmenter la profondeur d'une teinte noire. Cela montre l'intérêt de l'invention pour une utilisation dans des produits eyeliner et mascaras.

## Revendications

1. Utilisation cosmétique non thérapeutique d'une combinaison d'extrait de spiruline et/ou d'un pigment indigo et de pigment d'oxyde de fer noir pour apporter de l'éclat au teint de la peau et/ou des muqueuses.

2. Utilisation cosmétique non thérapeutique d'un extrait de spiruline et/ou de pigment indigo dans une composition cosmétique ou dermatologique comprenant un pigment d'oxyde de fer noir, pour augmenter la profondeur de la teinte noire de ladite composition cosmétique ou dermatologique dû à la présence du pigment d'oxyde de fer noir.

3. Utilisation cosmétique non thérapeutique selon la revendication 1 ou 2, dans laquelle ledit oxyde de fer noir est le tétraoxyde de fer, ledit extrait de spiruline provient d'au moins une espèce choisie parmi Spirulina Platensis et Spirulina Maxima, et ledit pigment indigo est un pigment choisi parmi un pigment naturel issu des feuilles et/ou tiges de l'indigotier (Indigofera Tinctoria) ou issu des feuilles et/ou tiges du Persicaire (Polygonum Tinctorium Leaf/Stem extract).

4. Association comprenant un extrait de spiruline et/ou de pigment indigo et un pigment d'oxyde de fer noir, dans laquelle le ratio entre la quantité d'extrait de spiruline ou d'extrait d'indigo et la quantité d'oxyde de fer noir est comprise entre 1/99 et 99/1, de préférence environ 100/0, la valeur de 0 étant possiblement exclue.

5. Association selon la revendication 4, dans laquelle ledit oxyde de fer noir est le tétraoxyde de fer, et ledit pigment indigo est un pigment naturel issu des feuilles et/ou tiges de l'indigotier (Indigofera Tinctoria) ou issu des feuilles et/ou tiges du Persicaire (Polygonum Tinctorium Leaf/Stem extract).

6. Composition cosmétique ou dermatologique comprenant une association selon la revendication 4 ou 5 et au moins un véhicule cosmétiquement et/ou dermatologiquement acceptable.

7. Composition selon la revendication 6, dans laquelle le pourcentage en poids de l'extrait de spiruline ou de pigment indigo est compris entre 0,0001 et 11,250% en poids, et le pourcentage en poids d'oxyde de fer noir est compris entre 0,0001 et 15,000% en poids, par rapport au poids total de la composition.

8. Composition selon la revendication 6 ou 7, **caractérisée en ce qu'**il s'agit d'une composition choisie parmi une composition unifiant le teint cutané, une composition de coloration des cils et/ou des sourcils, une composition soulignant le bord des paupières et une composition soulignant la partie mobile de la paupière.

9. Composition selon l'une quelconque des revendications 6 à 8, **caractérisée en ce qu'**elle est sous une forme choisie parmi une crème, un onguent, un baume, un beurre, un lait, une émulsion eau dans huile, une émulsion huile dans eau, un gel aqueux, et un produit anhydre sous forme de gel ou de stick.

10. Dispositif se présentant sous une forme choisie parmi un pot, un flacon-pompe, une lingette, un masque, un patch, un spray, un stick et une flaconnette, ledit dispositif comprenant une association telle que définie dans la revendication 4 ou 5, ou une composition telle que définie selon l'une quelconque des revendications 6 à 9.
